Europäisches Patentamt

European Patent Office

Office européen des brevets

(19)

(11) Numéro de publication: **0 206 942**
**A1**

(12) # DEMANDE DE BREVET EUROPEEN

(21) Numéro de dépôt: **86401363.6**

(22) Date de dépôt: **20.06.86**

(51) Int. Cl.⁴: **C 12 N 15/00,** C 07 H 21/04,
C 07 K 15/04, G 01 N 33/68

(30) Priorité: **21.06.85 FR 8509534**

(43) Date de publication de la demande: **30.12.86**
**Bulletin 86/52**

(84) Etats contractants désignés: **AT BE CH DE GB IT LI LU NL SE**

(71) Demandeur: **CENTRE NATIONAL DE LA RECHERCHE SCIENTIFIQUE (CNRS), 15, Quai Anatole France, F-75007 Paris (FR)**

(72) Inventeur: **Kohiyama, Masamichi, 7, rue P. Sémard, F-75009 Paris (FR)**
Inventeur: **Perbal, Bernard, 1, avenue Beethoven, F-78990 Guyancourt (FR)**
Inventeur: **Ben Mahrez, Kamel, 45, bld. Jourdan, F-75014 Paris (FR)**

(74) Mandataire: **Peaucelle, Chantal et al, S.C. Ernest Gutmann - Yves Plasseraud 67, boulevard Haussmann, F-75008 Paris (FR)**

(54) **Séquences d'ADN d'archaebactéries, homologues à l'oncogène v-myc, protéines codées par ces séquences, leurs procédés d'obtention et leurs applications immunologiques.**

(57) L'invention concerne des séquences d'ADN d'archae-bactéries capables de coder des protéines homologues à v-*myc* et les protéines correspondantes obtenues à partir d'extraits solubles d'archaebactéries. Ces protéines sont utilisées pour l'élaboration d'anticorps spécifiques utilisables notamment pour la détection in vitro de la présence de protéines codées par les oncogènes de famille *myc*.

EP 0 206 942 A1

Séquences d'ADN d'archaebactéries homologues à l'oncogène v-myc, protéines codées par ces séquences, leurs procédés d'obtention et leurs applications immunologiques.

L'invention a pour objet des séquences d'ADN d'archaebactéries homologues à l'oncogène v-myc, les protéines codées par ces séquences, leurs procédés d'obtention et leurs applications immunologiques.

Au cours de travaux antérieurs sur les enzymes des archaebactéries, les inventeurs se sont intéressés à la similitude des propriétés de ces bactéries avec celles des eucaryotes.

Il avait été en effet observé que l'ADN polymérase obtenue à partir des archaebactéries est pratiquement identique à celle des eucaryotes. De plus, le fractionnement de l'ADN-polymérase avait permis de mettre en évidence une activité reverse transcriptase comme dans les rétrovirus.

Une séquence homologue à l'oncogène v-myb, à l'origine de la myéloblastose, a été ensuite découverte par les inventeurs dans le génome d'archaebactéries telles que Halobacterium halobium.

L'état d'avancement de leurs travaux dans ce domaine les a amenés à rechercher l'existence chez Halobacterium halobium d'une séquence d'ADN homologue à l'oncogène v-myc responsable de la myélocytomatose.

L'invention repose sur la mise en évidence de la présence de cette séquence homologue chez plusieurs archaebactéries halophiles et méthanogènes.

L'invention a pour but de fournir des séquences d'ADN d'archaebactéries homologues à l'oncogène v-myc permettant de détecter l'activation de cet oncogène in vitro.

Elle vise également à fournir les protéines codées par ces séquences d'archaebactéries avec un degré de pureté élevée.

Elle vise en outre un procédé de purification de mise en oeuvre aisée des protéines codées par les séquences en question.

L'invention a également pour but de fournir des antisérums spécifiques vis-à-vis des protéines codées par les gènes de famille myc.

Les séquences d'ADN d'archaebactéries selon l'invention sont caractérisées en ce qu'elles s'hybrident avec la sonde de v-myc après traitement par l'endonucléase de restriction Xho I de l'ADN de l'archaebactérie.

La sonde v-myc est préparée selon P.H. Hamlyn et T.H. Rabbitts, dans Nature 304, 135-139, 1983.

L'archaebactérie peut être une archaebactérie halophile telle Halobacterium halobium ou méthanogène telle que Methanobacterium thermoautophicum (souche de Marburg). Halobacterium halobium est décrite notamment dans l'ouvrage de Bergey "Manual of determinative bacteriology", p. 272, 1974 et Methanobacterium thermoautophicum est décrite notamment dans Arch. Microb., 123, 105-107, 1979.

Dans le cas d'Halobacterium halobium après traitement de son ADN par l'endonucléase de restriction Xho I, on dispose de fragments de 3,4 kb et 2,3 kb environ.

Les protéines codées par les séquences de l'invention sont caractérisées en ce qu'il s'agit de protéines basiques, capables de réagir avec l'anticorps anti-myc (AB myc.1$^{TM}$). Cet anticorps est commercialisé par Oncor Inc., Gaithersburg, Maryland, U.S.A. 20877. AB myc.1$^{TM}$ réagit avec la portion spécifique de myc de la protéine p.110 de Δ gag-myc, cette protéine étant obtenue à partir de cellules aviaires transformées par le virus MC 29.

Le peptide codé par les séquences d'Halobacterium halobium possède un poids moléculaire d'environ 70 kDa.

D'une manière avantageuse, ces protéines sont présentes dans l'extrait brut soluble des archaebactéries, ce qui facilite leur purification en quantités importantes.

L'invention vise également les protéines purifiées correspondantes, ces protéines se présentant à l'état soluble.

On mesurera l'intérêt de l'existence de ces protéines à l'état soluble qui permet d'identifier plus aisément leurs fonctions enzymatiques et de préparer avec une plus grande efficacité des anticorps.

Conformément à l'invention, on sélectionne, de manière classique, par hybridation à l'aide d'une sonde marquée v-myc, un plasmide portant les séquences homologues à v-myc des archaebactéries.

Dans le cas d'Halobacterium halobium, on sélectionne un plasmide de 24 k paires de bases qui est constitué d'une part par une partie de génome de phage $\lambda$ et une partie de p$^{BR}$322 et d'autre part par la séquence homologue à v-myc de Halobacterium halobium.

Ce plasmide est utilisable comme sonde marquée permettant de détecter l'activation d'oncogènes myc.

Ce plasmide est amplifiable par la technique classique, ce qui facilite la préparation de l'ADN en quantités importantes.

Les protéines codées par ces séquences sont présentes, comme déjà indiqué, dans les extraits solubles bruts des archaebactéries.

Ces extraits solubles sont tout d'abord soumis à un traitement afin d'éliminer l'ADN présent.

Selon un mode de réalisation de l'invention, on

4

opère selon la technique classique de partition de phase en présence d'un sel d'environ 3 M.

Cette technique est avantageusement mise en oeuvre à l'aide de polyéthylèneglycol 6000 et de dextran T.500.

Selon un autre mode de réalisation, l'ADN est enlevé par précipitation à l'aide de streptomycine ou par une solution aqueuse de polyéthylèneimine (composé commercialisé sous la marque Polymin-P par BASF).

Les extraits d'archaebactéries dépourvus d'ADN ainsi obtenus sont traités afin d'éliminer au moins la majeure partie, pratiquement la totalité, des protéines acidiques.

A cet effet, on met avantageusement l'extrait brut en contact avec un échangeur d'ions chargés positivement. Cette opération est réalisée de préférence dans une colonne.

Avant d'effectuer l'étape de purification à l'aide des échangeurs d'ions, on élimine les sels présents dans les extraits. Cette élimination est effectuée de préférence par exemple par un traitement de dialyse. Il est avantageux d'effectuer cette dialyse en présence d'au moins un agent stabilisateur, par exemple du glycérol.

Pour les échangeurs d'ions chargés positivement pouvant être utilisés, on citera la DEAE-cellulose, DEAE-Séphacel ou la polyimine-cellulose (DEAE correspond au groupe diéthylaminoéthyle).

La fraction qui n'a pas été retenue sur la colonne est récupérée et mise en contact avec un échangeur d'ions chargé négativement.

On effectue avantageusement une chromatographie sur une colonne chargée avec par exemple un produit tel que l'hydroxyapatite.

Cette chromatographie est réalisée plus spécialement en présence d'au moins un stabilisateur et dans le cas de l'utilisation d'hydroxyapatite de tampon phosphate.

La récupération de la protéine recherchée est effectuée par élution à l'aide d'un tampon en gradient de concentration croissant.

Ces dispositions permettent d'obtenir des protéines de grande pureté correspondant à celles codées par les séquences homologues à v-myc et ce, de manière avantageuse, sous forme soluble.

En outre, cette préparation de protéine présente l'avantage d'une stabilité élevée.

Si on le souhaite, la purification de la protéine peut être poursuivie, par exemple, par chromatographie d'affinité par exemple dans une colonne d'ADN-cellulose ou de Bleu-Sépharose.

Selon un autre aspect de l'invention, l'antigénicité de la protéine obtenue est mise à profit pour l'élaboration d'anticorps spécifiques polyclonaux ou monoclonaux par injection selon les techniques classiques de ces préparations de protéine à des animaux et récupération des antisérums. Les anticorps sont récupérés par exemple par chromatographie d'affinité à partir de ces antisérums.

On notera l'intérêt de ces anticorps qui possèdent un large spectre d'action vis-à-vis des produits des gènes de la famille myc.

Ce large spectre d'action résulte de l'intégrité des séquences couvrant v-myc et également de la configuration tertiaire de la protéine de l'invention.

Or, jusqu'à présent, on ne connaissait que des anticorps spécifiques vis-à-vis de séquences partielles peptidiques codées par v-myc ou bien l'anticorps préparé par manipulation génétique, formé d'agrégats de peptides

v-myc difficiles à purifier en quantités importantes.

Les anticorps de l'invention peuvent être obtenus au contraire en quantités importantes et présentent un spectre d'action élargi.

Les antisérums et anticorps de l'invention constituent des réactifs de diagnostics particulièrement précieux, plus spécialement pour détecter in vitro des protéines codées par les oncogènes de famille myc présents dans les cultures de tissus de lignées de cellules cancéreuses par exemple. Il s'agit notamment de cellules cancéreuses de lymphomes de Burkitt ou bien de cellules cancéreuses d'ovaires.

Cette détection peut être effectuée par la technique immunologique classique de type ELISA et immuno Blotting.

D'autres caractéristiques et avantages de l'invention apparaîtront dans la description des exemples qui suivent.

Exemple 1 : Mise en évidence de séquences apparentées à myc dans Halobacterium halobium.

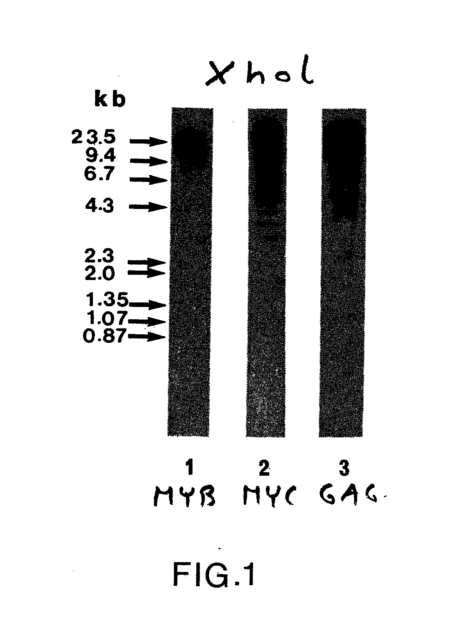

De l'ADN purifié d'Halobacterium halobium a été soumis à une digestion avec les endonucléases de restriction Xho I et les fragments obtenus ont été soumis à une électrophorèse, un blotting sur nitrocellulose et une hybridation avec des sondes marquées au $^{32}$P à translation de coupure (nick-translation) dans les conditions classiques. Comme le montre la figure 1, l'hybridation avec la sonde spécifique de HAX4 myb décrite par Perbal et coll. dans J. Virol. 44, 586-594 (1982), a conduit à la détection de fragments de 12 kb tandis que l'hybridation avec la sonde de myc (construite par Papas, (voir l'article dans Nature de 1983 mentionné dans la description)) a permis de mettre en évidence des fragments 5, 3,4 et 2,9 kb. Comme la sonde de myc qui a été utilisée contenait environ 1 kb du gène rétroviral gag, les produits de transfert par blotting ont été hybridés avec la sonde gag-spécifique dérivée d'ADN proviral MAVI, selon Soret et coll. dans J. Virol. sous presse. Après hybridation avec la sonde de gag, seul du fragment de 5 kb d'ADN a été détecté indiquant que les séquences apparentées à myc dans Halobacterium halobium étaient contenues dans les fragments 3,4 et 2,9 kb. Cette organisation précise des locus myc(H) et myb(H) dans l'ADN d'Halobacterium halobium et le degré d'homologie existant entre les gènes des archaebactéries et des eucaryotes ont été de plus déterminés par établissement de la carte et des séquences.

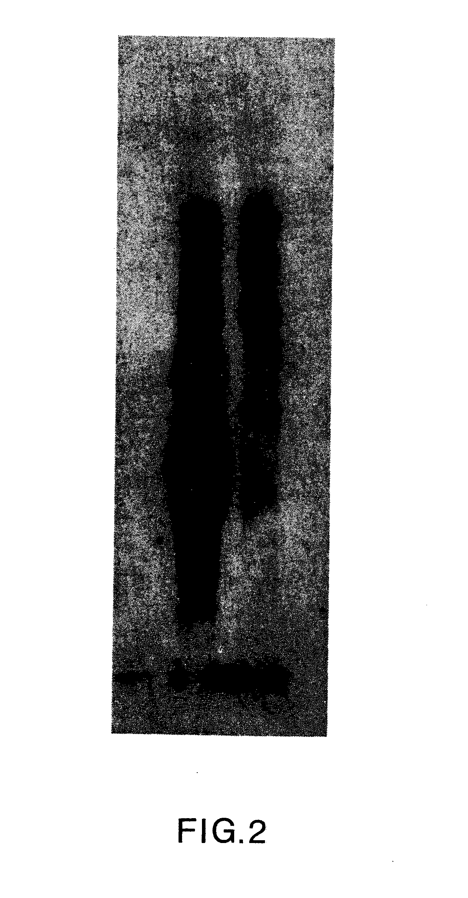

Pour déterminer si les gènes myc(H) et myb(H) sont exprimés dans Halobacterium halobium, de l'ARN de cellules en croissance exponentielle a été purifié selon la méthode de Chirgwin et coll. dans J. Biochem. 18, 5294-5299(1979), soumis à une électrophorèse dans les gels de formaldéhyde-agarose et transféré sur nitrocellulose. Les produits de transfert obtenus ont été hybridés soit avec la sonde myb-spécifique (HAX4) soit avec la sonde de myc. De plus, une hybridation témoin a été effectuée avec la sonde gag-spécifique (B2HI92). Les résultats obtenus (figure 2) ont révélé qu'aucune espèce d'ARN myb-spécifique ne peut être détectée dans Halobacterium halobium, tandis que deux espèces d'ARN différentes (3,8 et 1,9 kb) contenant les séquences de myc sont apparues exprimées dans ces cellules. Comme aucune de ces espèces d'ARN ne s'est hybridée avec la sonde gag-spécifique, il se révèle qu'elles représentent l'expression des séquences myc(H) présentes dans le génome d'Halobacterium halobium.

Exemple 2 : Etude de l'expression différentielle des séquences myb(H) et myc(H) au niveau des protéines correspondantes.

On utilise un extrait d'archaebactéries

contenant $10^9$ cellules par ml de culture, ces cellules étant en phase exponentielle de croissance. Pour préparer des extraits dépourvus d'ADN, on centrifuge puis on désintègre les cellules à l'aide d'un homogénéiseur du type Potter en présence de tampon et de KCl 3 M.

On élimine la matière à sédimentation rapide et on traite la fraction soluble selon la technique classique de partage de phase (dextran T 500-polyéthylèneglycol 6000), puis on dissocie les complexes protéine-ADN par KCl 3 M. La séparation électrophorétique des protéines d'Halobacterium halobium ainsi obtenues a été effectuée sur des gels de polyacrylamide dans des conditions provoquant une dénaturation. La présence de complexes immunitaires a été révélée avec le conjugué peroxydase-anti-IgG après transfert sur nitrocellulose et incubation en présence d'antisérums anti-myc, commercialisés par Oncor Inc., tels que définis ci-dessus ou anti-myb.

On remet en suspension 14 g d'Halobacterium halobium congelé dans 15 ml de tampon contenant 25 mM d'HEPES, 0,5 mM d'EDTA, 1 mM de dithiothréitol et 3 M de KCl et on désintègre avec un homogénéiseur Potter. Après centrifugation (100 000 g, 60 minutes), on aspire le surnageant et on ajoute 11 % de polyéthylèneglycol 6000 et 2,3 % de dextran T-500.

Après une heure d'agitation modérée, on centrifuge le mélange (5 000 g, 10 minutes) et on prélève la phase supérieure.

On précipite l'échantillon correspondant à 250 µg de protéines avec de l'ATC (acide trichloroacétique) (6 %) et on remet le culot en suspension, après lavage, dans un tampon sodium-phosphate 10 mM à pH 7, 1 % de SDS et 1 % de β-mercaptoéthanol, puis on chauffe à 100°C pendant 5 minutes.

On soumet l'échantillon à une électrophorèse sur gel de SDS-polyacrylamide. On transfère par électrophorèse les protéines du gel à une membrane de nitrocellulose à 50 V pendant 3 heures. Le tampon de transfert est constitué de 25 mM de Tris à pH 8,3, 192 mM de glycine et 20 % de méthanol.

Après le transfert, on lave la membrane pendant 2 heures à 37°C dans du tampon X (50 mM de Tris pH 7,5, 200 mM de NaCl, 0,1 % de Tween et 0,25 % de gélatine) et on incube pendant une heure à 37°C dans le même tampon contenant l'anticorps (dilution : 1/20).

On rince ensuite trois fois la membrane pendant 5 minutes dans le même tampon et on incube pendant une heure à 37°C avec du sérum $P_o$ de Miles (anti-IgG conjugué avec de la peroxydase) dans du tampon X (dilution : 1/1000).

Après trois rinçages, on révèle la membrane par incubation pendant 15 minutes dans du tampon contenant 15 mg de chloronaphtol, 5 ml de méthanol, 25 ml de (50 mM de Tris à pH 7,5 et 200 mM de NaCl) et 20 µl d'$H_2O_2$.

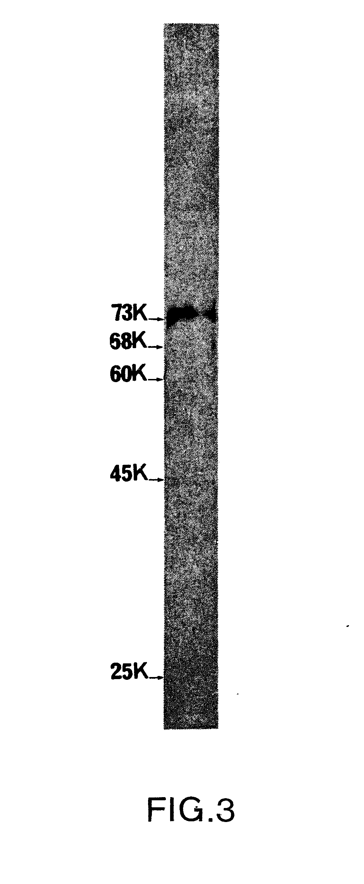

Comme le montre la figure 3, une bande unique de 73 kDa a été détectée avec l'antisérum anti-myc, tandis qu'aucune bande n'a été obtenue dans des conditions semblables avec l'antisérum anti-myb. Ces observations concordent avec les résultats obtenus dans les études d'hybridation effectuées avec de l'ARN purifié et indiquent que dans les cultures en croissance exponentielle d'Halobacterium halobium, les séquences de myc(H) sont exprimées alors que dans des conditions semblables, les séquences de myb(H) sont muettes.

Cette situation est comparable à ce que l'on a mentionné pour les cellules fibroblastiques d'origine aviaire dans lesquelles aucun produit apparenté à myb (ARN ou protéine) n'est exprimé tandis que des produits apparentés à c-myc peuvent être détectés dans l'ensemble du cycle cellulaire.

Exemple 3 : Purification de la protéine apparentée à v-myc.

Les étapes mises en oeuvre peuvent être réalisées à 5°C environ ou à température ambiante.

L'extrait dépourvu d'ADN tel qu'obtenu précédemment est dialysé pendant 2 heures contre 15 fois le volume de phosphate de sodium 20 mM (pH 7,0) contenant 100 mM de KCl, 0,5 mM de dithiothréitol et 35 % de glycérol (v/v).

On fait passer le dialysat à travers une colonne de DEAE-cellulose (Whatman) (5 x 1,5). La fraction non adsorbée contenant la protéine apparentée à v-myc est stabilisée immédiatement par addition de KCl 2 M. On fait passer cette fraction à travers une colonne d'hydroxyapatite (Bio Rad) (4,5 x 1,5) équilibrée préalablement à l'aide de 20 mM de phosphate de sodium (pH 6,8) contenant KCl 2 M et 10 % de glycérol. Après lavage de la colonne avec un tampon d'équilibration, on élue la protéine apparentée à v-myc à l'aide de phosphate de sodium 0,2 M (pH 6,8) contenant 10 % de glycérol.

Exemple 4 - Préparation d'un antisérum anti-myc.

La fraction du type v-myc obtenue est mélangée avec un adjuvant complet de Freud ou analogue et directement injectée à des lapins à raison de 1 ng de protéine par animal. L'injection est répétée un mois après et l'antisérum récupéré selon les techniques classiques.

REVENDICATIONS

1. Séquences d'ADN d'archaebactéries caractérisées en ce qu'elles s'hybrident avec la sonde de v-myc après traitement par l'endonucléase de restriction Xho I de l'ADN de l'archaebactérie.

2. Séquences selon la revendication 1, caractérisées en ce que l'archaebactérie est une archaebactérie halophile telle que Halobacterium halobium ou une archaebactérie méthanogène telle que Methanobacterium thermoautophicum.

3. Séquences selon la revendication 2, caractérisées en ce que, dans le cas du traitement de l'ADN d'Halobacterium halobium, le traitement par l'endonucléase de restriction Xho I conduit à des fragments de 3,4 kb et 2,9 kb environ.

4. Protéines correspondant à celles codées par les séquences homologues à v-myc, caractérisées en ce qu'il s'agit de protéines basiques, capables de réagir avec l'anticorps anti-myc (AB myc.1$^{TM}$), cet anticorps réagissant avec la portion spécifique de myc de la protéine p.110 de Δ gag-myc obtenue à partir de cellules aviaires transformées par le virus MC 29.

5. Peptide codé par les séquences d'ADN d'Halobacterium halobium, caractérisé en ce qu'il possède un poids moléculaire d'environ 70 kDa.

6. Protéines ou peptide selon la revendication 4 ou 5, sous forme purifiée, se présentant à l'état soluble.

7. Procédé d'obtention des séquences selon l'une quelconque des revendications 1 à 3, caractérisé en ce qu'on sélectionne, de manière classique, par hybridation à l'aide d'une sonde marquée v-myc, un plasmide portant les séquences homologues à v-myc des archaebactéries.

8. Procédé selon la revendication 7, caractérisé en ce que dans le cas d'Halobacterium halobium, on sélectionne un plasmide de 24 k paires de bases qui est constitué d'une part par une partie de génome de phage λ et une partie de p$^{BR}$322 et d'autre part par la séquence homologue à v-myc de Halobacterium halobium.

9. Procédé pour détecter l'activation d'oncogènes myc, caractérisé en ce qu'on utilise, comme sonde marquée, un plasmide tel que sélectionné dans le procédé selon la revendication 7 ou 8.

10. Plasmide de 24 k paires de bases constitué d'une part par une partie de génome de phage λ et d'une partie de p$^{BR}$322 et d'autre part par la séquence homologue à v-myc de Halobacterium halobium.

11. Procédé d'obtention d'une protéine selon l'une quelconque des revendications 4 à 6, caractérisé en ce que :

-on soumet les extraits solubles bruts des archaebactéries à un traitement afin d'éliminer l'ADN présent, tel que la technique de partition de phase en présence d'un sel d'environ 3 M, avantageusement à l'aide de polyéthylèneglycol 6000 et de dextran T.500,

-on ajoute, aux fins de précipitation de l'ADN, de la streptomycine ou une solution aqueuse de polyéthylèneimine telle que le composé commercialisé sous la marque Polymin-P,

-on élimine au moins la majeure partie, pratiquement la totalité des protéines acides par contact avec un échangeur d'ions chargés positivement tels que la DEAE-cellulose, le DEAE-Sephacel ou la polyimine-cellulose, les sels de l'extrait d'archaebactérie ayant été éliminés au préalable par exemple par dialyse, en présence d'un stabilisateur tel que le glycérol, récupération de la fraction qui n'a pas été retenue sur l'échangeur d'ions et mise en contact avec un échangeur d'ions chargé négativement, avantageusement avec une protéine telle que l'hydroxyapatite, de préférence en présence d'un stabilisateur et de tampon phosphate, récupération de la protéine recherchée par élution à l'aide d'un tampon de gradient de concentration croissante et poursuite, si on le souhaite, de la purification de la protéine, par exemple par chromatographie d'affinité notamment dans une colonne d'ADN-cellulose ou de Bleu-Sepharose.

12. Anticorps spécifiques polyclonaux ou monoclonaux tels qu'obtenus par injection, selon les techniques classiques, des préparations de protéines à des animaux et récupération des antisérums.

13. Réactifs de diagnostics élaborés à l'aide des anticorps et antisérums selon la revendication 12, pour détecter in vitro des protéines codées par les oncogènes de famille myc présents dans les cultures de tissus de lignées de cellules cancéreuses par exemple.

FIG.1

FIG.2

73K⟶
68K⟶
60K⟶

45K⟶

25K⟶

FIG.3

Office européen
des brevets

RAPPORT DE RECHERCHE EUROPEENNE

0206942
Numéro de la demande

EP  86 40 1363

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| Y | COMPTE RENDUS DE L'ACADEMIE DES SCIENCES DE PARIS, tome 300, série III, no. 5, 7 février 1985, pages 177-180, Académie des Sciences, Paris, FR; B. PERBAL et al.: "Existence de séquence homologues de l'oncogène V-MYB dans le génome des archaebactéries" * En entier * | 1-3 | C 12 N  15/00 C 07 H  21/04 C 07 K  15/04 G 01 N  33/68 |
| Y | NATURE, vol. 304, 14 juillet 1983, pages 135-139, Macmillan Journals Ltd.; P.H. HAMLYN et al.: "Translocation joins c-myc and immunoglobulin gamma 1 genes in a Burkitt lymphoma revealing a third exon in the c-myc oncogene" * En entier * | 1-3 | |

DOMAINES TECHNIQUES
RECHERCHES (Int. Cl.4)

C 12 N
C 12 P

Le présent rapport de recherche a été établi pour toutes les revendications

| Lieu de la recherche | Date d'achèvement de la recherche | Examinateur |
|---|---|---|
| LA HAYE | 19-09-1986 | CUPIDO M. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82